# FASCICULE DE BREVET EUROPEEN

(11) **EP 1 877 034 B1**
(45) Date de publication et mention de la délivrance du brevet: **27.04.2016**
(21) Numéro de dépôt: 06755466.7
(22) Date de dépôt: 28.04.2006
(51) Int. Cl.: A61K 31/352, A61K 8/97, A61P 17/12

(54) **UTILISATION DE POLYPHENOLS DE CACAO POUR LA REGULATION DU CYCLE CELLULAIRE DANS LE TRAITEMENT DES HYPERKERATINISATIONS**
VERWENDUNG VON KAKAOPOLYPHENOLEN ZUR REGULATION DES ZELLZYKLUS IN DER BEHNADLUNG DER HYPERKERATINISIERUNG
USE OF COCOA POLYPHENOLS FOR CELL CYCLE REGULATION IN THE TREATMENT OF HYPERKERATINISATION

(30) Priorité: 04.05.2005 FR 0504595
(43) Date de publication de la demande: 16.01.2008
(73) Titulaire: SOCIETE DE RECHERCHE COSMETIQUE SARL, 2314 Luxembourg (LU)
(72) Inventeur: LECLERE, Jacques, F-45500 Saint-gondon (FR)
(74) Mandataire: L'Helgoualch, Jean
(86) Numéro de dépôt international: PCT/FR2006/000966
(87) Numéro de publication internationale: WO 2006/117466

(56) Documents cités:
- FR-A- 2 810 242
- FR-A- 2 838 055
- DATABASE KOSMET [Online] ENNAMANY R. ET AL: "Active plant cells for the prevention of senescence and matrix degradation" XP002362280 extrait de STN Database accession no. 34820 & IFSCC CONFERENCE, "WORLD WIDE WELLNESS", 19-21 SEPTEMBER 2005, FLORENCE, ITALY, PROCEEDINGS, 2005, pages 1-8,
- WAROCQUIER-CLEROUT R ET AL: "NON-SAPONIFIABLE FRACTION OF COCOA SHELL BUTTER: EFFECT ON RAT AND HUMAN SKIN FIBROBLASTS" INTERNATIONAL JOURNAL OF COSMETIC SCIENCE, KLUWER ACADEMIC PUBLISHERS, DORDRECHT, NL, vol. 14, no. 1, 1992, pages 39-46, XP001031250 ISSN: 0142-5463
- R.R. LEATHERS ET AL: "The effect of different temperatures on teh growth, lipd conent and fatty acid composition of theobroma cacao cell suspension cultures" PLANT SCIENCE, vol. 62, no. 2, 1989, pages 217-227, XP002362231 cité dans la demande

## Description

La présente invention concerne une nouvelle utilisation de polyphénols extraits de cacao en cosmétique et en dermatologie, et plus particulièrement une nouvelle utilisation de polyphénols extraits de cacao pour la régulation du cycle cellulaire.

La peau constitue un véritable organe comprenant plusieurs couches intégrées, allant de la couche superficielle, l'épiderme, jusqu'aux couches plus profondes, le derme et l'hypoderme, et chacune possède des propriétés spécifiques permettant à l'ensemble de réagir et de s'adapter aux conditions de son environnement.

L'épiderme, principalement composé de kératinocytes (90% des cellules épidermiques), de mélanocytes (2 à 3% des cellules épidermiques) et des cellules de Langerhans, a une épaisseur variable selon les différentes parties du corps.

Le derme, plus épais, se compose principalement de collagène, d'élastine et de protéoglycanes. Ces trois types de molécules sont synthétisés par les fibroblastes dermiques. Les fibres de collagène assurent la résistance mécanique et la texture de la peau, l'élastine est responsable de l'élasticité, et les protéoglycanes jouent un rôle majeur de structure et d'hydratation de la peau. D'autres cellules comme les macrophages et les leucocytes sont également présentes dans la couche du derme.

L'hypoderme, qui est la couche la plus profonde de la peau, contient les adipocytes qui produisent des lipides pour que le tissu sous-cutané fabrique une couche grasse protégeant les muscles, les os et les organes internes contre les chocs.

Le vieillissement de la peau peut être intrinsèque, ou extrinsèque c'est-à-dire provoqué par l'environnement, y compris les agressions climatiques, qui peuvent notamment contribuer à accélérer la dégradation du collagène du derme, et en particulier l'exposition au soleil, les variations de températures et les radicaux libres. Les premiers signes du vieillissement de la peau, tels que les rides et ridules, sont généralement provoqués par le stress et les changements biologiques et physiologiques, accélérés par l'environnement extérieur ou par les modes de vie. L'apparition de marques pigmentaires, la diminution de l'épaisseur de la peau et son affaissement sont également des changements observés au cours du vieillissement. En fait, la capacité de la peau à remplacer le collagène endommagé diminue avec le temps, et des espaces et des irrégularités apparaissent dans le réseau du collagène. A l'échelle de la peau, le vieillissement provoque notamment une diminution des synthèses protéiques (collagène, élastine), une diminution de la synthèse des protéoglycanes entre autres, ainsi qu'une élévation des métalloprotéinases de type MMP3.

Dans les tissus normaux, existe un équilibre entre dégradation et synthèse tissulaire. Ainsi un excès de métalloprotéinases entraîne une dégradation de la matrice extra-cellulaire et de biomolécules telles que collagène, protéo-glycanne et gélatine, ce qui peut avoir des conséquences néfastes sur le tissu épidermique et peut aussi engendrer des maladies des cartilages, des processus inflammatoires, des mélanomes, etc.

Pour atténuer ou éliminer les signes du vieillissement de la peau, on a proposé divers traitements à base de compositions telles que crèmes et lotions contenant des alpha-hydroxyacides ou des rétinoïdes, appliquées régulièrement pour réduire progressivement le nombre de rides et ridules. On a aussi proposé des implants de collagène pour dissimuler les lignes d'expression autour des yeux ou de la bouche, la dermabrasion et les peelings chimiques pour éliminer la couche supérieure de la peau endommagée, la chirurgie esthétique, telle que la blépharoplastie (chirurgie des paupières) ou un lifting pour rajeunir une peau présentant un affaissement, ou encore une restructuration à l'aide d'un laser au dioxyde de carbone pour éliminer les ridules. Le brevet FR-A-2.783.169 décrit l'utilisation de pentapeptides du type Lys-Thr-Thr-Lys-Ser dans des compositions topiques pour favoriser la synthèse du collagène et des glycosaminoglycannes, et par conséquent la régénération cutanée.

Toutefois, si tous ces produits et méthodes connus peuvent avoir un effet favorable sur les signes du vieillissement cutané, par exemple en masquant ou en réduisant les rides, ils sont généralement sans incidence sur l'évolution cellulaire qui aboutit à ces signes du vieillissement.

On sait que les polyphénols d'origine végétale, extraits par exemple du raisin ou du cacao, ont des propriétés intéressantes utilisables en cosmétique. Par exemple, le brevet EP 1.289.491 enseigne que les polyphénols extraits du cacao (Theobroma cacao) possèdent une activité anti-ride utiles pour les soins contre le vieillissement de la peau. Le brevet FR 2.838.055 décrit l'utilisation d'extrait de cacao pour lutter contre l'hypersensibilisation cutanée et moduler la réponse inflammatoire de la peau en réaction à la présence de nickel.

Les polyphénols de cacao se répartissent en trois groupes qui appartiennent tous à la famille des flavonoïdes : les catéchines (flavanols, 37% env.) et notamment la (-)épi-catéchine, les procyanidines (oligomères flavanoliques, 58%) et les anthocyanes (5%), notamment le cynanidine-3-L-arabi-noside et le cyanidine-3-L-galactoside. Il a également été montré que des oligomères d'épicatéchine peuvent protéger les cellules et les tissus cutanés contre les effets des peroxynitrites qui sont des médiateurs de l'inflammation, en réagissant avec les peroxynitrites ou avec des produits de dégradation, jouant ainsi un rôle anti-inflammatoire. Des études réalisées sur des modèles animaux ont aussi montré que certains extraits de cacao enrichis en polyphénols pouvaient inhiber la promotion de tumeurs de la peau induites par le DMBA et le TPA, chez la souris [Osakabe et al., Am. Chem. Soc. 88-101 (1999)].

Aussi, bien que de nombreuses compositions dermatologiques et cosmétiques aient été proposées, il existe toujours un besoin de pouvoir disposer de nouvelles compositions topiques alternatives permettant d'agir sur les cellules cutanées, et notamment des compositions topiques à base d'extraits végétaux appropriés provenant plus particulièrement de plantes, capables de procurer des effets bénéfiques sur la peau, sans entraîner aucun effet toxique.

Malgré les progrès des techniques d'extraction végétale et de synthèse ou hémisynthèse organique permettant d'obtenir des produits identiques à ceux existant dans les plantes, l'intérêt des consommateurs pour les produits à base de plantes et de substances naturelles exige la mise au point de nouvelles techniques susceptibles de garantir la qualité et l'authenticité des produits.

Les études réalisées par la demanderesse ont montré que les polyphénols extraits du cacao pouvaient aussi avoir une influence sur le métabolisme cellulaire cutané et que les cellules de cacao obtenues par culture de cellules végétales de Theobroma cacao étaient particulièrement intéressantes pour la préparation de compositions cosmétiques et dermatologiques à cet effet.

Les extraits de cacao ont notamment été utilisés pour traiter les effets des agressions chimiques sur la peau (FR2838055) ou encore pour prévenir les signes du vieillissement de la peau (FR2810242).

La présente invention a donc pour objet une nouvelle utilisation de polyphénols extraits de cacao ou de cellules de cacao pour une utilisation comme médicament dermatologique destinée à réguler le cycle cellulaire cutané, chez des sujets affectés par une dégénérescence cellulaire se manifestant par une hyperkératinisation de la peau.

En particulier, il a été démontré que les polyphénols extraits du cacao ou de cellules de cacao permettent de lutter contre la sénescence cellulaire et de favoriser la régénération cellulaire.

L'invention a encore pour objet l'utilisation de polyphénols extraits du cacao ou de cellules de cacao pour la préparation d'une composition dermatologique permettant de lutter contre la dégénérescence cellulaire, ainsi qu'un procédé cosmétique pour combattre les effets de la dégénérescence cellulaire de la peau, consistant à appliquer sur les zones de la peau concernées une composition contenant une quantité efficace de polyphénols extraits du cacao ou de cellules de cacao, et ce pour l'effet décrit.

L'invention a encore pour objet une nouvelle composition topique à base d'extraits végétaux spécialement adaptée à une telle utilisation, comprenant un extrait de polyphénols de cacao ou des cellules de cacao en une concentration adaptée à l'effet recherché.

Les études effectuées ont montré l'intérêt des polyphénols de cacao, tant comme extraits de cacao (Theobroma cacao), éventuellement encapsulés, que comme cellules de cacao.

Ces études ont notamment montré, comme indiqué plus loin, que les polyphénols de cacao suivant l'invention
- présentent une parfaite innocuité vis-à-vis des cellules endothéliales de l'épiderme, les images histologiques des épidermes traités étant similaires à celles des témoins non traités ;
- entraînent le maintien des cellules cutanées en phase de croissance exponentielle se traduisant par un tapis cellulaire confluent et homogène.

Ainsi, les compositions topiques de l'invention peuvent être utilisées avantageusement en dermatologie pour assurer la régulation du cycle cellulaire chez des sujets susceptibles d'être affectés par une dégénérescence cellulaire se manifestant par une diminution des fonctions vitales des cellules cutanées, dans des situations d'hyperkératinisation indépendamment de l'âge de la personne.

Le cycle cellulaire cutané dure normalement environ 28 jours et comprend plusieurs phases successives, à savoir un intervalle entre la fin de la mitose et le début de la synthèse de l'ADN (phase G1), la phase de réplication de l'ADN (phase S), l'intervalle entre la fin de la réplication de l'ADN et le début de la mitose (phase G2) et la phase mitotique aboutissant aux deux cellules filles. En dehors de ce cycle cellulaire, les cellules peuvent se trouver en repos avec une diminution de l'activité métabolique (phase G0). La sénescence cellulaire se traduit essentiellement par un blocage des cellules dans la phase G1, sans passage à la phase de synthèse conduisant à la division cellulaire. Les cellules sénescentes sont caractérisées par un métabolisme actif avec surexpression de l'enzyme β-galactosidase. L'action des extraits de polyphénols de cacao, et des cellules de cacao, de l'invention permet d'éviter cette dégénérescence cellulaire, en régulant le cycle cellulaire et en augmentant le phase de quiescence, comme indiqué plus loin.

Les compositions selon la présente invention peuvent se présenter sous toutes les formes galéniques usuelles adaptées à une application topique, et de préférence sous forme de lotion, crème, lait ou sérum.

Elles peuvent comprendre entre 0,1% et 5% en poids d'extrait de polyphénols de cacao, par rapport au poids total de la composition, de préférence entre 0,2 et 1% en poids. Dans le cas de l'utilisation de cellules de cacao, les compositions peuvent comprendre entre 0,01% et 0,5% en poids de cellules de cacao, par rapport au poids total de la composition, de préférence entre 0,02% et 0,1% en poids.

Suivant une variante de la présente invention, il peut être avantageux d'utiliser des cellules de cacao obtenues par des techniques connues de croissance ou multiplication cellulaire, en milieu liquide ou solide, de cellules d'origine végétale cultivées *in vitro* dans un environnement contrôlé, dans des conditions aseptiques en l'absence de micro-organismes, au lieu d'utiliser des extraits de polyphénols de cacao.

La technique de la culture végétale présente l'avantage de produire des cellules cultivées dans des conditions environnementales bien définies et reproductibles, en ce qui concerne notamment la température, la lumière et le milieu de culture, et de produire des cellules non différenciées se trouvant au même stade physiologique à un instant donné. Un autre avantage est la formation de métabolites secondaires en un temps plus court, de l'ordre de une à trois semaines, que chez la plante où ce délai est de plusieurs mois. Ainsi, on pourra se référer aux travaux effectués sur des suspensions cellulaires de cacao tels que ceux de Gurney et Evans, Exp. Bot. 43, 769-775, (1992), Wen et Kinsella, J. Food Sc. 57, 1452-1457 (1992) et Leathers et Scragg, Plant Sc. 62, 217-228 (1989).

Suivant la présente invention, on peut plus particulièrement utiliser des cellules dédifférenciées lyophilisées de cacao dont les teneurs des trois principaux composants sont respectivement de 1,2 mg d'anthocyanes, 136,0 mg d'oligomères procyanidoliques (OPC) et 11,2 mg d'épicatéchine, par gramme de cellules lyophilisées. La lyophilisation des cellules permet d'améliorer leur conservation et, par suite, de faciliter leur incorporation dans les compositions cosmétiques.

Les compositions conformes à la présente invention peuvent être présentées sous les formes classiquement utilisées pour une application topique, c'est-à-dire sous forme de lotion, gel, émulsion (en particulier crème ou lait), masque, pommade, liposomes ou encore des patches transdermiques, contenant des excipients et supports usuels compatibles et pharmaceutiquement acceptables. Elles peuvent aussi se présenter sous forme de lingettes imbibées d'une solution contenant un extrait de polyphénols de cacao suivant l'invention.

Ces formes d'administration par voie topique sont préparées par les techniques usuelles, et par exemple, dans le cas d'une crème, par dispersion d'une phase grasse dans une phase aqueuse pour obtenir une émulsion huile dans eau, ou inversement pour préparer une émulsion eau dans huile. Dans le cas de crèmes, on préfère utiliser des émulsions à structure lamellaire contenant peu de produits éthoxylés ou n'en contenant pas du tout.

Les compositions topiques selon l'invention peuvent par exemple comprendre des excipients appropriés pour une administration topique externe, en particulier des excipients acceptables sur le plan dermatologique et cosmétologique. Ces excipients appropriés pour la formulation sont bien connus de l'homme du métier et comprennent en particulier des agents de pénétration tels que l'éthoxydiglycol, le phytantriol, l'octyl dodécanol et l'escine ; les épaississants tels que les gommes naturelles et les polymères de synthèse ; les émollients et les tensioactifs tels que l'octanoate de cétéaryle, le myristate d'isopropyle, l'isononanoate de cétéaryle, la diméthicone, la cyclométhicone, le 3-diisostéarate de polyglycéryle, le polyisobutène hydrogéné, l'alcool cétylique, le palmitate cétylique, le phosphate cétylique ; les émulsionnants tels qu'une lécithine ou un tensioactif à base d'inuline comme l'Inutec^{®} de la société Orafti ; les conservateurs tels que le phénoxyéthanol, le parahydroxybenzoate de méthyle (méthylparaben), le parahydroxybenzoate d'éthyle (éthylparaben), le parahydroxybenzoate de propyle (propylparaben) et le Phenonip® associant du phénoxyéthanol et des parahydroxybenzoates de méthyle, éthyle, butyle et isobutyle ; les colorants ; les parfums ; etc.

D'autres ingrédients peuvent être utilisés dans les compositions : les agents hydratants tels que le propylène glycol, la glycérine, le butylène glycol et également les vitamines antioxydantes telles que la vitamine E, par exemple l'acétate de tocophérol ou le tocotriénol, la vitamine C, les polyphénols naturels. On peut également ajouter à la composition des agents conditionneurs de la peau tels que le nylon et le nitrure de bore.

Il peut être avantageux, suivant une variante de la présente invention, d'incorporer dans la composition une substance présentant une activité complémentaire utile, par exemple une activité anti-élastase, ou une activité favorisant la synthèse du collagène. On peut utiliser par exemple le lipopeptide disponible dans le commerce sous la marque Matrixyl^{®} (Sederma) généralement sous forme de solution hydro-alcoolique titrée en palmitoyl-lysyl-thréonyl-thréonyl-lysyl-sérine.

Des agents de protection contre les rayons ultraviolets peuvent aussi être avantageusement incorporés dans les compositions, et par exemple des filtres solaires UV-A et UV-B hydrophiles ou lipophiles, choisis parmi la benzophénone ou un dérivé de benzophénone tel que la 2-hydroxy-4-méthoxy-benzophénone (Eusolex® 4360), ou un ester d'acide cinnamique et plus particulièrement le méthoxycinnamate d'octyle (Eusolex® 2292), le méthoxycinnamate d'éthyl-2-hexyle (Parsol MCX®), ou encore un cyano-β,β-diphénylacrylate tel que l'octo-crylène (Eusolex® OCR), le 4-méthylbenzylidène camphre (Eusolex 6300®), et des dérivés du dibenzoylméthane tels que le 4-isopropyl dibenzoylméthane (Eusolex 8020), le t-butyl-méthoxy dibenzoylméthane (Parsol 1789®), et le 4-méthoxy-dibenzoylméthane. On peut aussi utiliser des pigments formant écran anti-ultraviolet, comme par exemple le dioxyde de titane, l'oxyde de zinc, l'oxyde de zirconium ou encore l'oxyde d'aluminium.

Des exemples non limitatifs de compositions conformes à la présente invention sont donnés ci-après. Sauf indication contraire, les pourcentages et parties sont indiqués en poids.

### Exemple 1

Suivant les techniques classiques, on prépare un sérum de régénération cellulaire ayant la composition pondérale suivante.

| | |
|---|---|
| Eau déminéralisée | qsp 100,00 |
| EDTA trisodique | 0,10 |
| Pentylène glycol | 5,00 |
| Caprylyl glycol | 3,00 |
| Sorbate de potassium | 0,30 |
| Xylityl glycoside | 2,00 |
| Polyacrylate de sodium | 1,00 |
| α-tocophérol | 0,50 |
| Octyl dodécanol | 2,00 |
| Tetra-isopalmitate d'ascorbyle | 0,10 |
| Matrixyl^{®} | 3,00 |
| Essence d'ylang ylang | 0,10 |
| Palmitate de vitamine A 1M/UI | 0,20 |
| Polyphénols extraits de cacao | 0,50 |

L'extrait polyphénolique utilisé dans la composition ci-dessus est un extrait obtenu par broyage de fèves de cacao et séparation hydrophile/lipophile du beurre de cacao d'une part et d'un mélange de protéines et de polyphénols d'autre part. La séparation hydrophile / lipophile permet, par extraction de la graine broyée, de séparer à l'eau les corps gras surnageants de la phase polyphénols / protéines. On utilise de préférence une eau portée à une température comprise entre 80 et 100°C environ..

Le sérum ayant la composition indiquée ci-dessus est utilisé en application de préférence sur les bras et le visage, une à deux fois par jour pendant une période de 4 à 8 semaines.

### Exemple 2

Suivant une technique usuelle, on prépare une crème de régénération cellulaire de la peau ayant la composition pondérale suivante.

| | |
|---|---|
| Eau déminéralisée | qsp 100,00 |
| EDTA trisodique | 0,10 |
| Pentylène glycol | 5,00 |
| Caprylyl glycol | 3,00 |
| α-tocophérol | 0,70 |
| Octyl dodécanol | 2,00 |
| Palmitate de vitamine A 1M/UI | 0,20 |
| Tetra-isopalmitate d'ascorbyle | 0,10 |
| Huile d'onagre | 3,00 |
| Stéarate d'octyl stéaroyle | 3,00 |
| Huile d'arachide hydrogénée | 2,00 |
| Alcool béhénylique | 2,50 |
| Palmitate de cétyle | 1,50 |
| Hydroxyde de sodium | 0,10 |
| Phosphate d'alkyle C20-C22 | 2,00 |
| Beurre de Karité | 2,00 |
| Protéines de soja hydrolysées | 3,00 |
| Matrixyl^{®} | 3,00 |
| Cellules de cacao | 0,05 |

### Exemple 3

### Evaluation de la cytotoxicité

La cytotoxicité de l'extrait de polyphénols vis-à-vis des cellules endothéliales a été évaluée comme suit.

L'extrait polyphénolique de l'invention, à la concentration de 0,5%, et un placebo (même base sans l'extrait de polyphénol) sont déposés à raison de 10 µl/cm² sur des explants de peau humaine pendant 24 heures, comparativement à des explants témoins. Cet essai sur trois lots (un suivant l'invention, un témoin et un placebo) a été conduit en duplicate.

On constate que la composition de l'invention n'induit aucune toxicité.

Les images histologiques, après coloration HES des explants traités sont similaires à celles des explants témoins.

Ces résultats montrent que l'extrait de polyphénols selon l'invention ne présente aucune activité cytotoxique vis-à-vis des cellules endothéliales de l'épiderme, quelle que soit la concentration.

### Exemple 4

Evaluation de l'effet sur la sénescence cellulaire L'étude de l'action de la composition de l'invention sur la sénescence cellulaire de kératinocytes en culture par révélation de la β-galactosidase a été faite sur des kératinocytes d'origine humaine cultivés dans un milieu défini. Elle consiste en un marquage des cellules présentant la β-galactosidase, considérée comme biomarqueur de la sénescence cellulaire.

Suivant une méthode classique, on obtient des primo-cultures de kératinocytes à partir d'une biopsie de peau humaine. Les essais sont effectués sur les kératinocytes, entre le 8^{ème} et le 10^{ème} passage, afin d'assurer la présence des cellules sénescentes au niveau des cellules témoins.

Sachant que les cellules sénescentes surexpriment la β-galactosidase, la méthode consiste à fournir aux cellules le β-galactoside qui, après transformation, forme un précipité bleu significatif de l'activité de l'enzyme β-galactosidase.

Quatre lots ont été constitués :
- Lot 1 : témoin négatif ne recevant aucun produit.
- Lot 2 : traité par la composition à base de cellules de cacao à 0,02%.
- Lot 3 : traité par la composition à base de cellules de cacao à 0,05%.
- Lot 4 : traité par la composition à base de cellules de cacao à 0,10%.

L'essai est conduit en triplicate pour chaque lot.

Les kératinocytes sont ensemencés sur des lamelles dans des boîtes multipuits (6 puits) à raison de 10⁵ cellules par puits dans 3 ml de milieu de culture Skinethic^{®} supplémenté par l'EGF, l'hydrocortisone, l'insuline et la gentamycine. Ils sont ensuite maintenus en étuve sous CO₂ pendant 5 jours, en présence ou en l'absence de la composition de l'invention (cellules de cacao à différentes concentrations).

Après 5 jours d'incubation, les cellules ont été lavées avec un tampon phosphate puis fixées dans un mélange formaldéhyde (2%) - glutaraldéhyde (0,2%). Les tapis cellulaires ont été lavés trois fois avec un tampon phosphate puis mis en contact avec une solution colorante appropriée contenant du 5-bromo-4-chloro-3-indolyl-β-galactoside "X-Gal" (1 mg/ml).

Les échantillons sont conservés à 37°C en étuve pendant 6 à 12 heures. L'intensité de la coloration est évaluée sous microscope optique. Après coloration, les cellules ont été lavées à l'eau distillée puis séchées à 50°C.

Le résultat du comptage des cellules traitées aux différentes concentrations par rapport au témoin est indiqué dans le tableau ci-après.

| | Nb de cellules comptées | % de cellules sénescentes |
|---|---|---|
| Témoin négatif | 98 ± 16 | 24 |
| Cellules de cacao 0,02% | 105 ± 13 | ns |
| Cellules de cacao 0,05% | 131 ± 6 | 12 |
| Cellules de cacao 0,10% | 139 ± 15 | 10 |

Ces résultats confirment que la composition de l'invention, notamment sous forme de cellules de cacao, diminue significativement le taux de cellules sénescentes.

De plus, l'observation au microscope optique ces cellules traitées montre un maintien du taux de multiplication cellulaire, avec des cellules confluentes non individualisées, par rapport aux cellules témoins qui sont moins confluentes et comportent par endroit des cellules individualisées.

## Revendications

1. Composition dermatologique comprenant des polyphénols extraits de cacao (Theobroma cacao) ou des cellules de cacao pour une utilisation comme médicament dermatologique destiné à réguler le cycle cellulaire cutané chez des sujets affectés par une dégénérescence cellulaire se manifestant par une hyperkératinisation de la peau.

2. Composition selon la revendication 1, **caractérisée en ce que** la concentration en extrait de polyphénols de cacao est comprise entre 0,1% et 5% en poids par rapport au poids total de la composition.

3. Composition selon la revendication 1 ou 2, **caractérisée en ce que** la concentration en cellules de cacao est comprise entre 0,01% et 0,5% en poids par rapport au poids total de la composition.

4. Composition selon la revendication 2, **caractérisée en ce que** la concentration en extrait de polyphénols de cacao est de 0,5% en poids.

5. Composition selon la revendication 3, **caractérisée en ce que** la concentration en cellules de cacao est de 0,05% en poids.

## Patentansprüche

1. Dermatologische Zusammensetzung, umfassend Kakao (Theobroma cacao) extrahierte Polyphenole oder Kakaozellen für eine Anwendung als dermatologisches Arzneimittel, das bestimmt ist, den Zellzyklus der Haut bei Menschen zu regulieren, die unter einer Zelldegeneration leiden, die sich in Form einer Hyperkeratinisierung der Haut manifestiert.

2. Zusammensetzung nach Anspruch 1, **dadurch gekennzeichnet, dass** die Konzentration an Kakao-Polyphenolextrakt zwischen 0,1 % und 5 Gew.-% inklusive im Verhältnis zum Gesamtgewicht der Zusammensetzung beträgt.

3. Zusammensetzung nach Anspruch 1 oder 2, **dadurch gekennzeichnet, dass** die Konzentration an Kakaozellen zwischen 0,01 % und 0,5 Gew.-% inklusive im Verhältnis zum Gesamtgewicht der Zusammensetzung beträgt.

4. Zusammensetzung nach Anspruch 2, **dadurch gekennzeichnet, dass** die Konzentration an Kakao-Polyphenolextrakt 0,5 Gew.-% beträgt.

5. Zusammensetzung nach Anspruch 3, **dadurch gekennzeichnet, dass** die Konzentration an Kakaozellen 0,05 Gew.-% beträgt.

## Claims

1. A dermatological composition comprising polyphenols extracted from cocoa *(Theobroma cacao)* or from cocoa cells for a use as a dermatological drug intended to regulate the skin cell cycle in subjects affected with cell degeneration appearing as hyperkeratinization of the skin.

2. The composition according to claim 1, **characterized in that** the the concentration of cocoa extract of polyphenols is comprised between 0.1% 5% by weight based on the total weight of the composition.

3. The composition according to claim 1 or 2, **characterized in that** the cocoa cell concentration is comprised between 0.01% and 0.5% by weight based on total weight of the composition.

4. The composition according to claim 2, **characterized in that** the concentration of cocoa extract of polyphenols is all 0.5% by weight.

5. The composition according to claim 3, **characterized in that** the cocoa cell concentration is of 0.05% by weight.
